# EUROPEAN PATENT SPECIFICATION

(11) **EP 4 408 858 B1**
(45) Date of publication and mention of the grant of the patent: **11.03.2026**
(21) Application number: 22813336.9
(22) Date of filing: 30.09.2022
(51) Int. Cl.: C07K 14/37, A61K 47/64, A61P 35/00, A61K 38/00

(54) **RECOMBINANT GLYCAN BINDING PROTEINS AND ITS USE**
REKOMBINANTE GLYCANBINDENDE PROTEINE UND DEREN VERWENDUNG
PROTÉINES DE LIAISON AU GLYCANE RECOMBINANTES ET LEUR UTILISATION

(30) Priority: 01.10.2021 IN 202121044592
(43) Date of publication of application: 07.08.2024
(73) Proprietor: Unichem Laboratories Ltd, Mumbai, Maharashtra 400067 (IN)
(72) Inventor: SATHE, Dhananjay, Thane Maharashtra 400601 (IN); IYAPPAN, Saravanakumar, Hyderabad Telangana 500015 (IN); BAKSHI, Gautam, Ambala City Haryana 134003 (IN); PATIL, Ganesh, Navi Mumbai Maharashtra 410209 (IN)
(74) Representative: Marks & Clerk LLP
(86) International application number: PCT/IB2022/059341
(87) International publication number: WO 2023/053083

(56) References cited:
- WO-A1-2020/104911
- WO-A2-2010/095143
- LEONIDAS ET AL: "Structural Basis for the Carbohydrate Recognition of the Sclerotium rolfsii Lectin", JOURNAL OF MOLECULAR BIOLOGY, ACADEMIC PRESS, UNITED KINGDOM, vol. 368, no. 4, 17 April 2007 (2007-04-17), pages 1145 - 1161, XP022030023, ISSN: 0022-2836, DOI: 10.1016/J.JMB.2007.02.092

## Description

### Field:

The present disclosure relates to a recombinant glycan binding protein which is useful in cancer detection and therapy. The disclosure further relates to a pharmaceutical composition comprising the said lectin.

### Background:

Lectins are glycan binding proteins that are found and derived from a variety of living organisms including microorganisms, plants, and animals. Lectins exhibit high glycan / carbohydrate specificity and are known to agglutinate erythrocytes by virtue of their non-catalytic domain that binds reversibly to specific monosaccharides or oligosaccharides. They are known to have binding specificity towards the carbohydrate moieties on the surface of cells without altering the properties of the carbohydrates. This function finds many applications in diagnostic or assay techniques by way of detection of biomarkers, purification of glycoproteins, glycolipids and in cell selection processes.

Apart from diagnostics, lectins have been researched for exploring their usefulness as cancer therapeutics. Recently, numerous lectins have been explored for their ability to bind to cancer antigens selectively and exert various physiological effects such as apoptosis, cytotoxicity, proliferative/antiproliferative activities, metastasis, inhibition of cell adhesion, etc. as cancer cells exhibit varied changes in the carbohydrate structures on their cell surface.

Although, lectins from various sources exhibit high carbohydrate specificity but they differ in their physio-chemical properties such as molecular size and sugar specificities. Few plant lectins like Mistletoe lectins (MLs) are obtained from the diverse species of Viscum album L., sharing an extensive history of their use as potent anticancer agents in the prevention and treatment of various cancers. Mistletoe lectin-I (ML-I) is highly investigated among all MLs for the anti-proliferative activity that arises from its cytotoxic and immunomodulatory functions. However, using lectin from plant sources has its own inherent drawbacks such as a lack of selectivity, inconsistent quality, and difficulties in scaling up production at industrial scale.

US Patent No. 9500650 has disclosed usefulness of lectins in detecting the undifferentiation sugar chain marker by the lectin-lectin sandwich method, thereby enabling determination of presence or absence of differentiated cells. The lectins disclosed in said patent comprise of lectins derived from Sclerotium rolfsii, Coprinopsis cinerea, Agaricus bisporus, Xerocomus chrysenteron, Aleuria aurantia, etc using recombinant DNA technology. The said patent does not disclose any therapeutic ability of the lectins.

Tumour associated carbohydrate structures like Thomsen Friedenreich (TF) disaccharide (Galβ1-3GalNAcα1-Ser/Thr) and N-acetylgalactosamine (TN) monosaccharide (GalNAc) are found to be expressed on 90% of the cancerous cells. Sclerotium rolfsii lectin (SRL) exhibits specificity towards TF and TN antigens and is purified from the sclerotic bodies of the phytopathogenic fungus. Indian patent application No. 1265/MUM/2004 has disclosed binding specificity of SRL to a panel of human cell lines representing different types of cancers studied using flow cytometry analysis. However, the SRL lectin from natural sources affects yield, has purification process issues, thereby making the process highly expensive for mass production of the said lectin. Also, lectin derived from natural sources are encountered with solubility & stability issues further hindering the usability of the said lectins in diagnostics and therapeutics.

Further, protein synthesis is initiated by either methionine in eukaryotes or formylmethionine (N terminal methionine) in prokaryotes. During expression of recombinant proteins, N terminal methionine is cleaved by endogenous Methionine Aminopeptidase (MAP). The said cleavage process is not effective as quantity of recombinant protein expressed outperforms the capacity of limited amount of MAP to cleave the N terminal methionine, thereby a substantial amount of expressed protein contains methionine as first amino acid, which is not part of the mature protein. Further, proteins with N terminal methionine are prone to oxidation during production and storage, and this oxidation must be carefully watched for. Methionine sulfoxide or methionine sulfone can be produced when methionine residues in proteins are oxidised, which may further result in increased immunogenicity, inactivity, and aggregation; thereby limiting the therapeutic product's clinical efficacy, stability and regulatory acceptance.

The biological products having N terminal methionine impurities may not have the same structure as that of the protein in the human body; and hence when administered in a human subject may result in unexpected immune response from the subject resulting in ineffective therapeutic functions. Therefore, it is important that the methionine impurity should be removed or minimized from the biological product before formulation.

Indian granted patent No. 277986 (appl. # 350/MUM/2009) has disclosed a modified recombinantly expressed lectin derived from native Sclerotium Rolfsii lectin. The said patent further discloses a method for preparing a recombinant lectin expressed in a host cell such as E.coli or yeast. Further, method comprises the synthesis of a lectin gene based on amino acid sequence derived from the MALDI MS/MS of the Sclerotium Rolfsii lectin, a soil borne fungus, and its cloning in the host cell. The lectin as mentioned in said patent usually comprises of the mixture of lectin with and without N terminal Methionine which may lead to hurdles in regulatory approval process. The lectin protein may also tend to form multimers, especially dimers due to availability of cysteine residues in the amino acid sequence.

Patent WO2020044296 disclosed that increased solubility of expressed protein in a host cell may alter or reduce affinity towards antigen binding specificity of the protein. Hence, it is important that the recombinant proteins must exhibit sufficient stability and show solubility while retaining the binding affinity towards specific antigen.

Patent WO2020074977 disclosed a method of preparing a recombinant lectin protein having less than 20% of recombinant lectin with initiator methionine. The said patent utilizes upstream and downstream strategy to reduce the expression of lectin with N terminal methionine variant/impurity.

Also, new therapeutic concepts like targeted drug therapies are recently under development, Protein Drug Conjugates (PDC) wherein antibody or protein is conjugated to a potent drug molecule with cytotoxic activity, optionally via a chemical linker.

An ideal PDC has a high specificity towards an antigen which is absent in a normal (healthy) cell; potent cytotoxic agent (generally a small molecule drug with high systemic toxicity) designed to induce target cell death after being internalized in the tumour cell; and optionally a chemical linker that is stable in circulation but releases the cytotoxic agent in target cells. However, use of a chemical linker necessitates use of additional chemical processes and the downstream purification. Also, most of the antibodies have ability to bind to antigens specifically expressed on particular type of cancer cell. To date, Antibodies having specificity towards a universal antigen represented on majority of cancer cell is not reported. Hence, proteins having high specificity towards a universal antigen represented on majority of cancer cell are under scientific investigation. Such protein when conjugated to potent drug molecules may pave the way for a universal therapy for effective treatment of numerous types of cancers.

Various conjugation chemistries have been used in past for preparation of protein drug conjugates. Few of the reported conjugation chemistries include Lysine Conjugation Chemistry, Maleimide Protein Conjugation Reaction Chemistry, etc. For preparation of antibody drug conjugates, lysine-based conjugation process is one of the most preferred process due to its versatility and simple process requirement and can react with several cytotoxic agents (especially onto antibodies). But with proteins, wherein lysine amino acid residues are present at multiple locations, the said process may result into generating heterogeneous molecule. And the heterogenous molecule requires tedious purification and characterization process, thereby resulting into regulatory approval hurdles.

A stable and soluble protein enabling easy conjugation ability to make protein drug conjugates with improved homogeneity for preparation of biopharmaceutical drugs, having ability to deliver the conjugated agent to a target location i.e., cancer cell, is desired.

WO 2010/095143 A2 describes recombinant lectins which bind to glycans on cancer cells.

WO 2020/104911 A1 describes biopharmaceutical combinations of a recombinant lectin protein and other therapeutic agents.

Leonidas et al (Journal of Molecular Biology, Academic Press, Vol. 368, No: 4, pages 1145-1161) describes the structural basis for the carbohydrate recognition of the Sclerotium rolfsii Lectin.

### Summary:

In a first aspect, the present invention provides a recombinant glycan binding protein comprising a variant of Sclerotium rolfsii Lectin (SRL) represented by SEQ ID NO: 1, wherein, as compared to SEQ ID NO: 1, the variant comprises:
a) an additional cysteine at amino acid position 142 at the carboxy-terminal end; or an additional serine at amino acid position 142 and an additional cysteine at amino acid position 143 at the carboxy-terminal end;
b) Serine in place of threonine at amino acid position 1; and
c) Glycine in place of Cysteine at amino acid position 76;

wherein the variant retains binding affinity towards one or more antigens selected from Thomsen-Friedenreich (Galβ1-3GalNAcα-O-Ser/Thr, TF or T) antigen, O-GalNAc Core1 (T antigen), Core2, 'α2,3/6-sialy1 Core1' (Sialyl-T antigen), 'α2,6/6-sialyl Core2' and modified forms of TF and T antigens,
and wherein the amino acid sequence of the said variant has at least 90% sequence identity with SEQ ID NO: 1.

In a second aspect, the present invention provides a pharmaceutical composition comprising: a recombinant glycan binding protein as described above; and one or more pharmaceutically acceptable excipient(s).

In a third aspect, the present invention provides a recombinant polynucleotide encoding a recombinant glycan binding protein as described above.

In a fourth aspect, the present invention provides a cell transfected with the recombinant polynucleotide as described above.

gThe amino acid modifications of the recombinant glycan binding protein variant of Sclerotium Rolfsii Lectin (SRL) described above result in attributes including enhanced molecule stability, reduced methionine impurities, aiding in conjugation to other biological and chemical molecules/agents; providing stability to the protein by avoiding cysteine-cysteine dimerization; and facilitating efficient removal of N-terminal methionine.

### Figures:

FIG. 1: Solubility Analysis of expressed protein of SEQ ID NO: 3
FIG. 2: Solubility Analysis of expressed protein of SEQ ID NO: 4
FIG. 3: Solubility Analysis of expressed protein of SEQ ID NO: 3 during fermentation stage
FIG. 4: Solubility Analysis of expressed protein of SEQ ID NO: 4 during fermentation stage
FIG. 5: Colocalization study of SEQ ID NO: 2

### Definitions:

The term "amino acid" as used herein refers to naturally occurring and synthetic amino acids, as well as amino acid analogues and amino acid mimetics that have a function that is similar to the naturally occurring amino acids. Naturally occurring amino acids are those encoded by the genetic code and include the proteinogenic amino acids. Naturally occurring amino acids also include those modified after translation in cells. Synthetic amino acids include non-canonical amino acids such as selenocysteine and pyrrolysine. Typically, synthetic amino acids are not proteinogenic amino acids.

The term "protein" as used herein refers to a polymer of amino acid residues.

The term "recombinant" means a nucleic acid, or a polypeptide that has been artificially or synthetically (i.e., non-naturally) altered by human intervention. The alteration can be performed on the material within, or removed from, its natural environment or state. For example, a "recombinant nucleic acid" is one that is made by recombining nucleic acids, e.g., during cloning, DNA shuffling or other well-known molecular biological procedures. A "recombinant DNA molecule" is comprised of segments of DNA joined together by means of such molecular biological techniques.

The term "recombinant protein" or "recombinant polypeptide" as used herein refers to a protein molecule which is expressed using a recombinant DNA technology. The recombinant protein may be expressed using site directed mutagenesis. Site-directed mutagenesis (SDM) is a method to create specific, targeted changes in double stranded plasmid DNA. These specific alterations, insertions, deletions or substitution is used to change or modify the protein structure or the activity. Methods of site-directed mutagenesis have evolved rapidly since the initial description of this concept. Smith, M., Annv. Rev. Genet. 19, 423-462 (1985). A common feature of the available methods is the use of synthetic oligonucleotides carrying the desired changes in the nucleotide sequence at the site of mutagenesis. This "mutant" oligonucleotide is incorporated into the sequence of interest by replacing the normal sequences with the designed oligonucleotide. This is accomplished by in vitro enzymatic DNA synthesis. The modified DNA is transformed to appropriate host system for expression of encoded protein(s).

The term "lectin" or "lectin protein" as used herein refers to a glycan/carbohydrate binding protein of Sclerotium Rolfsii (a soil borne pathogenic fungus of Indian origin), having National Center for Biotechnology Information (NCBI) Accession Number 2OFC_A, unless otherwise indicated by context.

The term "variant" as used herein refers to a polymer of amino acid residues that has set of highly similar proteins that originate from a single gene or gene family and are the result of genetic differences. They usually have a similar structure and functionality. A variant usually contains at least one amino acid modification as compared to the native protein.

The term "substitution" as used herein refers to the replacement of an amino acid at a particular position in an amino acid sequence with any other suitable amino acid.

The term "Cytotoxic agent" refers to a substance that inhibits cells or inhibits the function of cells and/or causes destruction of cells. The term is intended to include chemotherapeutic agents, toxins such as small molecule toxins or enzymatically active toxins of bacterial, fungal, plant or animal origin, including synthetic analogues and derivatives thereof.

The term "Conjugate" as used herein refers to the products of the present disclosure. For the purpose of present disclosure, the conjugate may comprise an agent attached to the protein with/without the aid of a linker, or it may comprise an agent directly attached to protein.

The terms "cancer" and "cancerous" refer to or describe the physiological condition or disorder in mammals that is typically characterized by unregulated cell growth.

The terms "treat" or "treatment," unless otherwise indicated by context, refer to therapeutic treatment and prophylactic measures to prevent relapse, wherein the object is to inhibit or slow down (lessen) an undesired physiological change or disorder, such as the development or spread of cancer. For purposes of this disclosure, beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Those in need of treatment include those already with the condition or disorder as well as those prone to have the condition or disorder.

The term "excipient" as used herein refers to inactive or usually inert substances that are added to the formulation which do not affect the therapeutic action of the active ingredient but serves as the vehicle or medium for the active ingredient. It may be used to provide a desired consistency, to improve stability, and/or to adjust osmolality of the composition. For the purpose of this disclosure excipients used are, but not limited to, protein stabilizing agents, buffers, polymers, solubilizers, Cryoprotectants, diluents or mixture thereof.

The term "medicament", as used herein, means a pharmaceutical formulation containing at least one pharmaceutically acceptable active compound. The physical state of the medicament includes but is not limited to liquids, solids, semi-solids, suspensions, powders, pastes, gels, and the like, and combinations thereof

The term "sequence identity" as used herein refers to the occurrence of exactly the same nucleotide or amino acid in the same position in aligned sequences.

The terms 'formulation', 'pharmaceutical formulation' and 'pharmaceutical composition' are used interchangeably and refer to preparations which are in such a form as to permit the biological activity of the active ingredients to be effective, and therefore may be administered to a subject for therapeutic use, wherein the subject is preferably human, unless otherwise indicated by context.

The term "Targeted delivery" refers to a system of specifying the agent directly into its targeted body area (organ, cellular, and subcellular level of specific tissue) to show its activity. This is done in order to overcome the aspecific & undesirable effect of agent, thereby reducing the amount of agent required for intended effectiveness.

The term "thiol-maleimide reaction" is a simple & rapid reaction between a thiol and a maleimide to generate a thiosuccinimide product and is used for site-selective modification of cysteine residues in bioconjugation technology. The thiol-maleimide reaction is used to add chemical labels onto biomolecules via thiol conjugation such as fluorescent dyes, PEG, radiolabels, and small molecules.

### Detailed Description:

The present invention provides a recombinant glycan binding protein comprising a variant of Sclerotium rolfsii Lectin (SRL) represented by SEQ ID NO: 1, wherein, as compared to SEQ ID NO: 1, the variant comprises:
a) an additional cysteine at amino acid position 142 at the carboxy-terminal end; or an additional serine at amino acid position 142 and an additional cysteine at amino acid position 143 at the carboxy-terminal end;
b) Serine in place of threonine at amino acid position 1; and
c) Glycine in place of Cysteine at amino acid position 76;

wherein the variant retains binding affinity towards one or more antigens selected from Thomsen-Friedenreich (Galβ1-3GalNAcα-O-Ser/Thr, TF or T) antigen, O-GalNAc Core1 (T antigen), Core2, 'α2,3/6-sialy1 Core1' (Sialyl-T antigen), 'α2,6/6-sialyl Core2' and modified forms of TF and T antigens,
and wherein the amino acid sequence of the said variant has at least 90% sequence identity with SEQ ID NO: 1.

In an aspect of the present disclosure, the recombinant glycan binding protein which is a variant of Sclerotium Rolfsii Lectin (SRL) represented by SEQ ID NO. 1, is designed and expressed in a suitable host cell.

In the present invention, the amino acid sequence of the said variant has at least 90% sequence identity with SEQ ID NO. 1. In a more particular embodiment, the sequence identity of the variant is at least 95%, 96%, 97%, 98% or 99% as compared to Sclerotium Rolfsii Lectin (SRL) represented by SEQ ID NO. 1.

In the present invention, the recombinant glycan binding protein is a variant of Sclerotium rolfsii Lectin (SRL) represented by SEQ ID NO: 1 as described herein. In one embodiment, the said variant retains binding affinity compared with the wild-type Sclerotium rolfsii Lectin (SRL) represented by SEQ ID NO. 1. The said binding affinity is envisaged to be toward one or more antigens selected from Thomsen-Friedenreich (Galβ1-3GalNAcα-O-Ser/Thr, TF or T) antigen, O-GalNAc Core1 (T antigen), Core2, 'α2,3/6-sialyl Core1' (Sialyl-T antigen), 'α2,6/6-sialyl Core2' and modified forms of TF and T antigens such as and altered expression of branched and fucosylated *N*- and O-glycans, including changes in Lewis antigens (SLe^{x} and SLe^{a}) which are expressed in more than 90% of human cancers. In addition to alterations in core glycans, each of these carbohydrates can be further modified to generate unique terminal glycan motifs that may also undergo specific changes following neoplastic transformation. For example, highly fucosylated glycans, such as Lewis antigens [Lewis^{a/b} (Le^{a/b}) and Lewis^{x/y} (Le^{x/y})], can become enriched on the cell surface following neoplastic transformation. Similarly, sialylation, a common terminal glycan modification, can also undergo significant changes during neoplastic progression.

In one embodiment of the present disclosure, variants or modified forms of TF antigen (or T antigen) includes [3OSO3]Galβ1-3GalNAcα; Neu5Acα2-3Galβ1-3 [6OSO3] GalNAca; Neu5Acα2-3Galβ1-3GalNAcα; GlcNAcβ1-3Galβ1-3GalNAcα; Galβ1-3(Galβ1-4GlcNAcβ1-6) GalNAcα; Galβ1-3(Neu5Acα2-3Galβ1-4GlcNAβ1-6)GalNAc; Galβ1-3(GlcNAcβ1-6)GalNAcα; Galβ1-3(Neu5Acα2-6)GalNAcα; Neu5Acα2-3Galβ1-3(Neu5Acα2-6)GalNAcα; Galβ1-4GlcNAcβ1-6(Galβ1-3) GalNAcα; Galβ1-3GalNAcα (TF antigen); Neu5Acα2-6 (Gal(31-3)GalNAca; Galβ1-3(Galβ1-4GlcNAcβ1-6)GalNAc; Galβ1-3(GlcNAcβ1-6)GalNAc; GlcNAcβ1-6 (Galβ1-3) GalNAcα; Galβ1-3 (Neu5Acβ2-6)GalNAcα; Fucα1-2Galβ1-3GalNAcα; Neu5Acβ2-6 (Galβ1-3)GalNAcα; GlcNAcβ1-2Galβ1-3GalNAcα.

In an embodiment of the present disclosure, the recombinant glycan binding protein which is a variant of Sclerotium rolfsii Lectin (SRL) represented by SEQ ID NO. 1 as described herein comprises a further alteration of protein by insertion, deletion, or substitution of an amino acid in SEQ ID NO: 1 such that said alteration does not alter the structural and functional activity of the protein insofar as the protein/variant retains binding affinity toward one or more antigens selected from Thomsen-Friedenreich (Galβ1-3GalNAcα-O-Ser/Thr, TF or T) antigen, O-GalNAc Core1 (T antigen), Core2, 'α2,3/6-sialy1 Core1' (Sialyl-T antigen), 'α2,6/6-sialyl Core2' and modified forms of TF and T antigens.

In an embodiment of the present disclosure, the recombinant glycan binding protein which is a variant of Sclerotium rolfsii Lectin (SRL) represented by SEQ ID NO. 1 as described herein, and compared to the wild-type SRL, is devoid of amino acid Cysteine (C) at amino acid positions between 1 and 141 of SEQ ID NO: 1. The said variant is prepared by insertion, deletion, or substitution of an amino acid in SEQ ID NO. 1 such that said alterations does not alter the structural and functional activity of the protein, namely, the protein/variant retains binding affinity toward one or more antigens selected from Thomsen-Friedenreich (Galβ1-3GalNAcα-O-Ser/Thr, TF or T) antigen, O-GalNAc Core1 (T antigen), Core2, 'α2,3/6-sialy1 Core1' (Sialyl-T antigen), 'α2,6/6-sialyl Core2' and modified forms of TF and T antigens.

In the present invention as described herein, the recombinant glycan binding protein which is a variant of Sclerotium rolfsii Lectin (SRL) represented by SEQ ID NO: 1, compared to the wild-type SRL, comprises an additional Cysteine at carboxy terminal end. The addition of Cysteine at carboxy terminal end is done in such a manner that it does not alter the structural and functional activity of the protein, namely, the protein/variant retains binding affinity toward one or more antigens selected from Thomsen-Friedenreich (Galβ1-3GalNAcα-O-Ser/Thr, TF or T) antigen, O-GalNAc Core1 (T antigen), Core2, 'α2,3/6-sialyl Core1' (Sialyl-T antigen), 'α2,6/6-sialyl Core2' and modified forms of TF and T antigens.

In an embodiment of the present disclosure, recombinant glycan binding protein of SEQ ID NO: 2 is a variant of wild-type Sclerotium Rolfsii Lectin (SRL), designed and expressed in a host cell. A recombinant glycan binding protein of SEQ ID NO: 2 may be further modified to obtain a recombinant glycan binding protein of the present invention, as described below. The said recombinant glycan binding protein of SEQ ID NO: 2 has been disclosed in Indian Patent Application 350/MUM/2009. The protein of SEQ ID NO: 2 has two carbohydrate binding sites (Primary & Secondary) having affinity towards GalNAc and GlcNAc glycans; and in one embodiment, the protein of SEQ ID NO. 2 has high binding affinity towards the oncofetal Thomsen-Friedenreich (Galβ1-3GalNAcα-O-Ser/Thr, TF or T) antigen and its derivatives, which are expressed in more than 90% of human cancers. The oncofetal Thomsen-Friedenreich (Galβ1-3GalNAcα-O-Ser/Thr, TF or T) antigen and its derivatives includes O-GalNAc Core1 (T antigen); and extended core structures comprising of Core2, α2,3/6-sialyl Core1 (Sialyl-T antigen), α2,6/6-sialyl Core2, and modified forms of TF and T antigens.

Recombinant glycan binding protein of SEQ ID NO. 2 when expressed in a suitable host such as E coli, expressed protein comprises of ~10-30 % N terminal methionine impurities. The said proteins with N terminal methionine are prone to oxidation during production and storage. Methionine oxidation may limit the therapeutic product's clinical efficacy, stability and regulatory acceptance. In an embodiment of the present disclosure, to overcome the aforementioned issue, the amino acid at position 1 may be substituted with a suitable amino acid, which aids in efficient removal of N terminal methionine using endogenous system of the host cell. It is very well known to person skilled in the art, that substitution may be done in such a manner that it does not affect the structure and function of the said protein. In a particular embodiment, Threonine (T) at position 1 of SEQ ID NO: 2 may be substituted with a suitable amino acid, which aids in efficient removal ofN terminal methionine using host cell processing mechanism. In a more particular embodiment, Threonine (T) at the first position of SEQ ID NO: 2 may be substituted with amino acid Serine (S), aiding in efficient removal of N terminal Methionine at the zero position. The said substitution is done in such a manner that the functional stability and binding affinity of the protein towards TF and TN antigen is not affected.

In an embodiment of the present disclosure, variants of Sclerotium rolfsii Lectin (SRL) represented by SEQ ID NO. 1 which are prepared as per the aforementioned embodiments are envisaged to be further conjugated to an agent. The said agent may be selected from a group comprising of therapeutic agent, cytotoxic agent, radioactive agent, anti-cancer agent, diagnostic agent, and combinations thereof.

For preparation of the conjugates, the agent may be reacted with protein using any suitable conjugation chemistries known in the art. The use of random conjugation chemistry may lead to generation of heterogenous conjugate molecules. The said heterogeneity may further lead to characterization, efficacy and safety issue in patients. To avoid similar issues, site specific conjugation of drugs to protein may be preferred/designed. In an embodiment, the recombinant glycan binding protein of SEQ ID NO. 2 may be re-engineered to make use of site-specific conjugation of drugs phenomenon. In a more particular embodiment, site specific conjugation chemistry used is a thiol-maleimide reaction chemistry i.e., cysteine-based site-specific conjugation. To harness the advantage of cysteine-based site-specific conjugation, SEQ ID NO. 2 may be re-engineered to comprise Cysteine (C) at carboxy terminal end of SEQ ID NO: 2 i.e. at 142^{nd} position, however Cysteine (C) at 142^{nd} position may make disulfide bond with Cysteine (C) at 76^{th} position leading to changes in protein structure due to scrambling, further leading to difficulties in purification/downstream process development. Also, changes in protein structure may lead to changes in structural and functional bioactivity. The said issues may be addressed by substitution of Cysteine (C) amino acid (available at 76^{th} position of SEQ ID NO. 2) with any other suitable amino acid. The said substitution may be done in such a manner that the functional stability and binding affinity of the protein towards Thomsen-Friedenreich (Galβ1-3GalNAcα-O-Ser/Thr, TF or T) antigen, O-GalNAc Core1 (T antigen), Core2, 'α2,3/6-sialy1 Core1' (Sialyl-T antigen), 'α2,6/6-sialyl Core2' and modified forms of TF and T antigens is not affected. In a particular embodiment, the Cysteine (C) amino acid available at 76^{th} position of SEQ ID NO. 2 is substituted with Glycine (G).

Having regard to the modifications described above, in an embodiment of the present disclosure, recombinant glycan binding protein of SEQ ID NO. 2 is re-engineered to have modifications at 1^{st}, 76^{th} and 142^{nd} positions to obtain a recombinant glycan binding protein according to the present invention. The said modification comprises substituting Serine at 1^{st} position for Threonine; substituting Glycine at 76^{th} position for Cysteine; and addition of Cysteine at carboxy terminal end of the SEQ ID NO. 2. In another embodiment of the present disclosure, re-engineered recombinant glycan binding protein is a protein of SEQ ID NO. 3.

In an embodiment of the present disclosure, recombinant glycan binding protein of SEQ ID NO: 2 is re-engineered to have modifications at 1^{st}, 76^{th} and 142-143^{rd} positions to obtain a recombinant glycan binding protein according to the present invention. The said modification comprises substituting Serine at 1^{st} position for Threonine; substituting Glycine at 76^{th} position for Cysteine; and addition of Serine at 142^{nd} position; and Cysteine at 143^{rd} position i.e., carboxy terminal end of the SEQ ID NO. 2. In another embodiment of the present disclosure, re-engineered recombinant glycan binding protein is a protein of SEQ ID NO. 4. The addition of Serine at 142nd position may aid in providing the space and flexibility between the c-terminal cystine and the protein, further providing flexibility to the conjugated drug and avoiding solubility issue with the protein conjugate.

In another embodiment of the present disclosure, protein of SEQ **ID** NO. 3 or SEQ **ID** NO. 4 may be further used for preparation of protein conjugates, more particularly using thiol-maleimide reaction to provide homogenous mixture of conjugates. The said homogenous conjugates having controlled Drug-Antibody Ratio (DAR) further resulting in conjugates with improved therapeutic index.

In an embodiment of the present disclosure, nucleic acid sequence of SEQ ID NO. 5 and SEQ ID NO. 6 may be used for expression of recombinant glycan binding protein of SEQ ID NO. 3 and SEQ ID NO. 4, respectively.

In an embodiment of the present disclosure, the variants of Sclerotium rolfsii Lectin (SRL) represented by SEQ ID NO. 1 and the conjugates prepared using the said variants are for use in diagnosis or therapeutic treatment of cancer.

In an embodiment of the present disclosure, a pharmaceutical composition comprising variants of Sclerotium rolfsii Lectin (SRL) represented by SEQ ID NO. 1 or the conjugates prepared using the said variants as described herein; in the presence of one or more pharmaceutically acceptable excipient(s) is provided. The pharmaceutically acceptable excipient(s) may be selected to provide support or enhance stability, bioavailability, or patient acceptability. The said pharmaceutical composition may be provided in a pharmaceutically acceptable form, such as a liquid (e.g in an aqueous solution or suspension, or as an oil based solution or suspension.), a solid (e.g a capsule or tablet), a lyophilized powder, a spray, cream, lotion or gel, vesicular drug delivery systems such as, but not limited to, bilosomes, liposomes, niosomes, transferosome, ethosomes, sphingosomes, pharmacosomes, multilamellar vesicles, microsphere and the like.

Various aspects of the disclosure are further described by way of below listed examples. The examples are illustrious, and the skilled person is well aware and informed about the obvious variations of them. The examples therefore do not limit the scope of this disclosure in any manner.

### Examples:

### Example 1: Manufacturing process of recombinant proteins

### Expression of recombinant glycan binding protein of SEQ ID NO. 3 and SEQ ID NO. 4

The nucleotide sequence (SEQ ID NO: 5) coding for the recombinant glycan binding protein of SEQ ID NO. 3 was cloned into pET27b vector using NdeI and BamHI to generate pET27b construct. Further the pET27b construct was transformed into propagation host E. coli Top 10 competent cells. Plasmid was isolated form transformed cells and insert integrity was confirmed by restriction digestion analysis and the gene sequence is confirmed by DNA sequencing. Verified pET27b plasmid was further transformed into E. coli BL21 DE3 (Gold) expression host. Transformed clones were tested for recombinant protein expression and the clone showing high expression is verified by DNA sequencing and further used for cell bank preparation. Characterized cell bank is used in fermentation process.

For fermentation seed culture was prepared by inoculating the culture from glycerol stock in medium containing yeast extract (10 g/L), potassium dihydrogen phosphate (3 g/L), dipotassium hydrogen phosphate (12.54 g/L), ammonium sulphate (5 g/L), sodium chloride (0.5 g/L), dextrose (12g/L), magnesium sulphate heptahydrate (1 g/L), kanamycin sulphate (20 mg/L) and trace metal solution (1 ml/L). The fermentation was initiated at 30°C and later the temperature was gradually decreased to 18°C. DO level was maintained at 60% and pH was maintained at 6.80±0.05 with alkali solution except during carbon source shifting. Fed batch fermentation process was used with glycerol (50%) and yeast extract (40%) as carbon and nitrogen source respectively as feed medium. Induction was done with 0.15 mM IPTG at OD600nm around 60. Total batch run time was 48 to 72 hours and maximum OD600 achieved was in the range of 107 after 52 log hours. Expression of the recombinant protein having amino acid sequence of SEQ ID NO 3 was around 6.3 g/L. Fermentation broth was harvested from the fermenter. It is then centrifuged at 9000 rpm, for 10-15 minutes at 10 °C to obtain compact cell pellet. Pellet obtained is then suspended in 1:10 ratio (w/v) of lysis buffer containing 25 mM Tris HCl, 1 mM EDTA, pH 8.5 and suspension is stirred for 1-2 hours using mechanical stirrer maintaining a temperature of 10±4°C. Lysis is carried out using high pressure homogenizer at 1100±200 bar by giving 1-2 passes. The cell lysis efficiency of >90-95% is obtained (determined by OD600) and lysate collect at low temperature.

The cell lysate was clarified using 0.1µ TFF and permeate was collected. In order to recover maximum quantity of protein, the retentate was washed 4-6 times with 25 mM Tris-HCl buffer, 1mM EDTA, pH 8.0 in step mode till protein absorbance at 280 nm came down to less than 4-6. The solution was later clarified using 0.1µ hollow fiber filter with Trans Membrane Pressure of 2-10 psi. The washes that contained the protein were pooled with the main permeate. Temperature was maintained below 25 °C during entire clarification process.

The protein of SEQ ID NO. 3 was purified using three step chromatography. In first step cell lysate was loaded on Cellufine Max Q resin pre equilibrated with 25 mM Tris buffer containing 1 mM EDTA, pH 8.0±0.5 and washed with the 25 mM Tris HCl, 1mM EDTA, 50 mM NaCl at pH 8.0. Protein was eluted with 25 mM Tris HCl, 1mM EDTA, 200 mM NaCl at pH 8.0. Entire peak was collected as a single fraction. In second step the protein of SEQ ID NO. 3 eluted from first step was bound to the CM Sepharose column pre equilibrated with the equilibration buffer at a binding capacity of 20 mg/ml. Post complete loading, column was washed with the equilibration buffer 25mM Sodium Acetate, 5mM β- Mercaptoethanol, pH 5.1 to remove the unbound protein. Elution was carried out giving a linear gradient from 0-50% 25mM Sodium Acetate, 1.0M NaCl, 5mM β- Mercaptoethanol, pH 5.1 for 15 column volumes followed by a wash of 100% buffer 25mM sodium scetate, 1.0M NaCl, 5mM β-Mercaptoethanol, pH 5.1. Elute obtained from CM Sepharose column then subjected to pH adjustment to 8.0 with Tris buffer and buffer exchanged against 25mM Tris, 5mM Beta mercaptoethanol, pH 8.0 to get conductivity to less than 2.0 mS/cm using 5KDa membrane. The protein of SEQ ID NO. 3 was loaded in third column Source 30Q pre-equilibrated with equilibration buffer: 25 mM Tris, 5mM Beta mercaptoethanol, pH 8.0. The protein of SEQ ID NO. 3 was then eluted using elution buffer of 25 mM Tris, 5mM Beta mercaptoethanol, 500mM NaCl, pH 8.0. The recombinant protein of SEQ ID NO. 3 with purity in excess of 95% was obtained.

Similarly, protein of SEQ ID NO. 4 was prepared with purity in excess of 95%.

The molecular mass and pI of purified protein of SEQ ID NO. 3 and SEQ ID NO. 4 were found to be as given below

| Protein | Molecular Mass | pI |
|---|---|---|
| SEQ ID NO. 3 | 16086.75 | 6.49 |
| SEQ ID NO. 4 | 16173.83 | 6.49 |

### Example 2: Cytotoxicity (MTS-PMS) assay of expressed protein.

Bioactivity of the protein of SEQ ID NO. 3 and SEQ ID NO. 4 was assayed using MTS-PMS bioassay. Effect of the said proteins on Ovarian Cancer cell line PA-1, Blood Cancer Cellline MOLT-3, Blood cancer cellline Jurkat E6, and Breast cancer celline MDA-MB 231 was studied.

5000 cells/well were used for testing.

The drug Doxorubicin was used as system suitability (SST) reference.

### Results of the cytotoxicity assay were as follows:

| **Table No. 1 - MTS - PMS Bioassay** | | | | | |
|---|---|---|---|---|---|
| **Drug Substance** | | **% Cytotoxicity on Cell-lines** | | | |
| **Test Sample** | **Concentration** | **MDA-MB 231** | **PA-1** | **MOLT-3** | **Jurkat E6** |
| Doxorubicin | 100 µm | 45% | - | - | - |
| SEQ ID NO. 2 | 80 µg/ml | 71% | 81% | 46% | 61% |
| SEQ ID NO.3 | 80 µg/ml | 56% | 82% | 43% | 68% |
| SEQ ID NO. 4 (reducing) | 80 µg/ml | 53% | 77% | 46% | 62% |
| SEQ ID NO. 4 (non-reducing) | 80 µg/ml | 56% | 72% | 45% | 55% |

### Conclusion:

- Proteins of SEQ ID NO. 2, 3, and 4 showed better cytotoxicity results as compared to Doxorubicin drug on MDA-MB-231 cells.
- Proteins of SEQ ID NO. 2, 3, and 4 showed better cytotoxicity results as compared to Doxorubicin drug on other cell line i.e., PA-1, MOLT-3, & Jurkat E6 cell-lines.
- The proteins showed similar bioactivity and hence can be inferred to have similar functionality.

### a. Comparative Example 3: Glycan binding study and co-localization study Glycan binding study was performed to understand the binding affinity and specificity of the Lectin having SEQ ID NO 2 towards glycans expressed on cancer cells.

Lectin of SEQ ID NO 2 was assayed with following glycan arrays for identifying specific binding motifs:
- O-Glycan array containing 94 glycans; and
- N-Glycan array containing 100 glycans

### Observations:

- The Lectin having SEQ ID NO: 2 showed specific binding to N-glycans containing GlcNac at the non-reducing end; estimated Kd with terminal N-glycan GlcNAc motifs were between 0.238 to 0.460 Kd (ug/ml).
- Lectin having SEQ ID NO: 2 showed strong and broad binding profile to O-glycans, specifically to T-antigen and its extended core structures. Main binding motifs found were O-GalNAc Core1 (T antigen) ; Extended Core1 structures; Core2; α2,3/6-sialyl Core1; and α2,6/6-sialyl Core2. Estimated Kd for T antigen is 0.319, Sialyl T antigen is 0.670 to 1.756 and for core 2 is 0.212 Kd (ug/ml).

### b. Colocalization study of SEQ ID NO 2

Study was performed to analyze the localization of SEQ ID NO 2 (test item) in cell organelles using Confocal microscopy.

Test Item provided as aqueous solution was tagged with FITC label with protein concentration of 1mg/mL.

The stock solution of Test Item was diluted in Serum Free Medium (SFM) at single concentration- 100µg/mL.

Cells were plated on a glass coverslip at a density of 0.1x10⁶cells per well. After complete adhesion of the cells on the coverslip, cells were treated with 100µg/mL of FITC- Recombinant Lectin having amino acid sequence of SEQ ID NO: 2 for 2 hours at 37°C.

Following the incubation cells were washed with PBS (3 washes) and fixed using 4% paraformaldehyde for 10 min at RT. After fixation, cells were permeabilized using 0.5% IGEPAL for 10 min at RT (Permeabilization was not done for cells that were stained with Pan-Cadherin). Post permeabilization, cells were blocked using 5% normal goat serum for 1 hour at RT. Post blocking, cells were treated with organelle specific markers for 1.5 hours at RT followed by secondary antibody for 2 hours at RT.

Following antibodies were used for different organelles:

**Table 3: Antibodies used for organelles**

| **Organelle** | **Organelle Specific marker** | **Dilution** | **Secondary antibody** | **Dilution** |
|---|---|---|---|---|
| Mitochondria | MTCO2 | 1: 50 | Texas red Goat Anti-mouse IgG | 1:500 |
| Endoplasmic reticulum | Calnexin | 1: 50 | Anti-mouse Texas red | 1:500 |
| Golgi bodies | GM130 | 1: 50 | Anti-mouse Texas red | 1:500 |
| Nuclear Envelope | Lamin | 1: 50 | Anti-mouse Texas red | 1:500 |
| Plasma Membrane | Pan Cadherin | 1: 50 | Anti-rabbit alexa fluor red | 1:500 |

Post incubation, cells were washed using PBST (3 washes) and PBS (3 washes) and stained with DAPI (nucleus stain) for 3 min at RT.

Cells were washed (3 washes) and mounted on a glass slide using Prolong Glass antifade mountant. Imaging was done using Confocal fluorescence microscope (Make: Olympus FV1000). Images were captured using software FluoView.

To determine the co-localization of recombinant lectin having amino acid sequence of SEQ ID NO:2 (recombinant protein), bladder cancer cells (T24) were incubated with recombinant lectin having amino acid sequence of SEQ ID NO:2 labelled with FITC tag for 2 h. This was followed by staining with organelle specific markers- MTCO2 (Mitochondria), Calnexin (Endoplasmic reticulum), GM130 (Golgi bodies), Pan Cadherin (Plasma membrane) and Lamin A/C (Nuclear Membrane). Cells were imaged using confocal microscope.

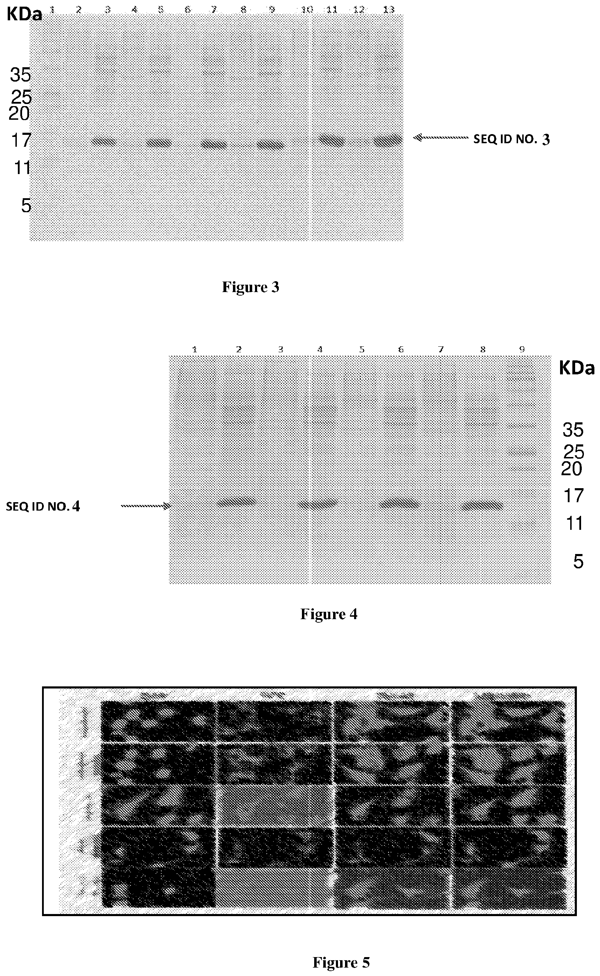

Results demonstrated that Recombinant Lectin having amino acid sequence of SEQ ID NO:2 (Recombinant Protein) (Recombinant Protein) colocalized in Mitochondria, Endoplasmic reticulum, Golgi bodies and Plasma membrane (red fluorescence). [see Fig 5].

### Example 4: Comparison of expressed protein with regards to N terminal Methionine

N terminal methionine content of the protein of SEQ ID NO. 2, 3 and 4 was measured using Reverse Phase - High Performance Liquid Chromatography.

| **Test Sample** | **Purity (%)** | **N Terminal Methionine (%)** |
|---|---|---|
| SEQ ID NO. 2 | 86.29 % | 13.18 % |
| SEQ ID NO. 3 | 98.21 | 1.07 % |
| SEQ ID NO. 4 | 95.72 % | 1.25 % |

Conclusion: Protein of SEQ ID NO: 3 and 4 showed less than 2% N terminal methionine impurity, whereas Protein of SEQ ID NO: 2 showed more than 13% N terminal methionine.

### Example 5: Comparison of expressed protein with respect to stability

Stability of expressed protein i.e., SEQ ID NO. 3 was evaluated for 2 weeks using 10 mM TBS buffer with pH 7.8 at 2-8 °C .

| **Timepoints** | | | | | | | | |
|---|---|---|---|---|---|---|---|---|
| **0 Week** | | | **1 Week** | | | **2 Week** | | |
| % purity | % single max impurity | % Total impurity | % purity | % Single max impurity | % Total impurity | % purity | % Single max impurity | % Total impurity |
| 98.05 | 1.31 | 1.95 | 97.34 | 0.98 | 2.66 | 97.09 | 0.86 | 2.91 |

Conclusion: The expressed protein i.e., SEQ ID NO. 3 was found to be stable in 10 mM TBS buffer with pH 7.8 at 2-8 °C.

### Example 6: Comparison of expressed protein with respect to solubility

Solubility of expressed protein of SEQ ID NO. 3 and 4 in production medium (induced at 18 °C with 0.25 mM IPTG) was evaluated using SDS PAGE analysis.

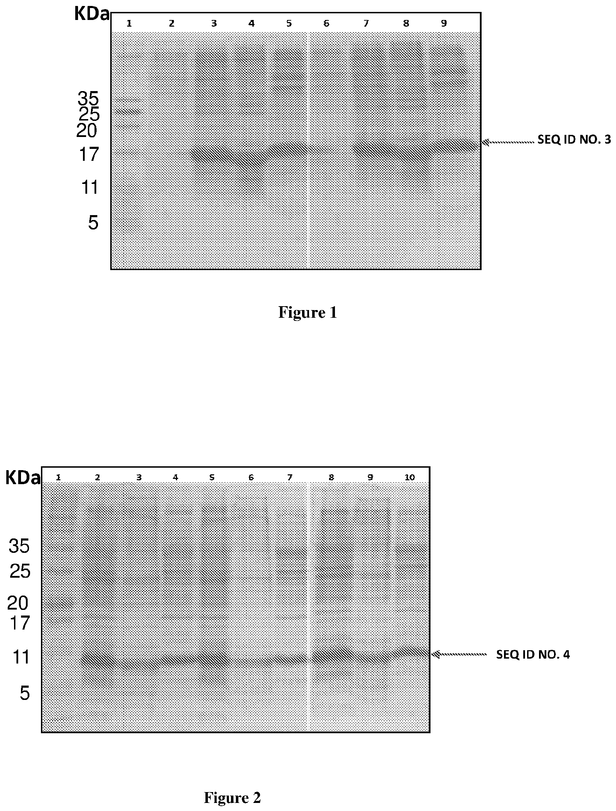

For results, refer Figure 1 and Figure 2.

| **Figure 1**: Solubility of expressed protein of SEQ ID NO. 3 | | |
|---|---|---|
| **Lane** | **Samples** | **Band%** |
| 1 | marker | - |
| 2 | batch 1- 0 hour | - |
| 3 | batch 1-overnight | 56.0 |
| 4 | batch 1-pellet | 49.6 |
| 5 | batch 1-supernatant | 47.2 |
| 6 | batch 2- 0 hour | - |
| 7 | batch 2- overnight | 52.5 |
| 8 | batch 2-pellet | 51.1 |
| 9 | batch 2 supernatant | 55.4 |

| **Figure 2****: Solubility of expressed protein of SEQ ID NO. 4** | | |
|---|---|---|
| **Lane** | **Samples** | **Band%** |
| 1 | marker | - |
| 2 | batch 1-overnight | 15.64 |
| 3 | batch 1-pellet | 18.14 |
| 4 | batch 1-supernatant | 20.41 |
| 5 | batch 2- overnight | 19.72 |
| 6 | batch 2-pellet | 16.40 |
| 7 | batch 2 supernatant | 18.49 |
| 8 | batch 3- overnight | 16.57 |
| 9 | batch 3pellet | 10.44 |
| 10 | batch 3- supernatant | 14.55 |

**Conclusion:** Solubility of expressed proteins of SEQ ID NO. 3 and 4 was confirmed in production medium.

### Example 7: Expression analysis of Soluble proteins i.e., SEQ ID NO. 3 and 4 at different timepoints during fermentation stage.

The cells were grown in fermentation medium under controlled conditions and induced with suitable inducer. Harvesting was done at different timepoints and analysed using SDS-PAGE for expression of soluble proteins.

| **Solubility of expressed protein of SEQ ID NO. 3** | | | |
|---|---|---|---|
| **Lane** | **Samples** | **Abs. Quant. (µg/10uL)** | **Relative %** |
| 1 | Marker | - | - |
| 2 | Log 45 hrs Pellet | 0.33 | 6.6 |
| 3 | Log 45 hrs supernatant | 4.66 | 93.4 |
| 4 | Log 48 hrs Pellet | 1.52 | 29 |
| 5 | Log 48 hrs supernatant | 3.71 | 71 |
| 6 | Log 51 hrs Pellet | 0.40 | 8.5 |
| 7 | Log 51 hrs supernatant | 4.29 | 91.5 |
| 8 | Log 54 hrs Pellet | 1.30 | 23 |
| 9 | Log 54 hrs supernatant | 4.32 | 77 |
| 10 | Log 57 hrs Pellet | 1.73 | 26 |
| 11 | Log 57 hrs supernatant | 4.87 | 74 |
| 12 | Log 60 hrs Pellet | 1.84 | 24 |
| 13 | Log 60 hrs supernatant | 5.81 | 76 |

| **Solubility of expressed protein of SEQ ID NO. 4** | | | |
|---|---|---|---|
| **Lane** | **samples** | **Abs. Quant. (ug/10uL)** | **Relative %** |
| 1 | Log 45 hrs Pellet | 0.34 | 8.8 |
| 2 | Log 45 hrs supernatant | 3.51 | 91.2 |
| 3 | Log 48 hrs Pellet | 0.22 | 5.7 |
| 4 | Log 48 hrs supernatant | 3.58 | 94.2 |
| 5 | Log 51 hrs Pellet | 0.19 | 3.9 |
| 6 | Log 51 hrs supernatant | 4.63 | 96.0 |
| 7 | Log 54 hrs Pellet | 1.31 | 23.2 |
| 8 | Log 54 hrs supernatant | 4.33 | 76.8 |
| 9 | Marker | - | - |

**Conclusion:** Solubility of expressed proteins of SEQ ID NO. 3 and 4 was confirmed in production medium during fermentation stages at various timepoints. See Fig. 3 and 4.

### Example 8: Preparation of Protein Drug Conjugate

Protein of SEQ ID NO. 3 was conjugated to cytotoxic drug MMAE using below procedure: 30 mg of protein of SEQ ID NO. 3 in 1X PBS (7.0 mg/ml) was diluted to final concentration 2.5 mg/ml by 1X PBS with 10 mM EDTA, DMA and 3.0 eq of 10 mM MC-Val-Cit-PAB-MMAE in DMA that resulted in a final DMA concentration of 20% (V/V). After 2 hours the reaction was incomplete and another 3.0 eq of MC-Val-Cit-PAB-MMAE was added and incubated for 2 h.

The protein drug conjugate was subjected to LCMS analysis to determine the DAR value. The product formed was subjected to size exclusion chromatography (SEC) using GE10-300 Superdex 75 column with buffer exchange to 50mM Tris, 150 mM NaCl and pH was adjusted to 8.0 using TBS.

Post SEC purification DAR value of protein drug conjugate was determined to be 1.0.

**Conclusion:** The protein drug conjugate prepared using Protein of SEQ ID NO. 3 were determined to have DAR value 1, which further suggested the homogeneity of the conjugate formed.

### Example 9: Bioassay - Cytotoxicity effect of conjugate

Bioassay - Cytotoxicity effect Conjugate of Example 8 against ovarian cancer cell line (PA-1) and human urinary Bladder cancer cell line (T-24) was assayed.

5,000 cells/well were plated and incubated at 37°C overnight.

Conjugates/vehicle control dilutions were made in respective media and added to cells. Volume added: 50uL to each well. Starting treatment concentration was 1 µM for Conjugate of example 8, and control SEQ ID NO: 3 alone and 10% for vehicle control (1X PBS with 20% DMA) and then diluted 10-fold down for a total of 11 treatment dilutions. Each concentration was analysed in triplicates. Media-only wells was used as a control to calculate percent viability. Cell titer glow reagent (volume: 50uL) was added to each well, the plate was shaken for 5 mins and the luminescence was recorded. Drug Treatment Time: 72 hours. Percent viability was calculated by dividing the luminescence signal obtained for each treated well by the untreated well (media-only control) and multiplying by 100.

Data was next transformed using X= Log (x) and then analysed with nonlinear regression (curve fit), Dose Response inhibition - log (inhibitor) vs response (3 parameters) using PRISM software to determine the IC50 value.

Results obtained were as follows:

| **Compound** | **IC50 values** | |
|---|---|---|
| | **PA-1 Cell line** | **T-24 cell line** |
| Conjugate of Example 8 | 17.7 nM | 325.4 nM |
| SEQ ID NO: 3 | 16µg/ml | - |

**Conclusion:** Conjugated molecule showed very high Potency as compared to SEQ ID No.3 on the cell line PA-1 and T-24. IC50 values are in the rage of 15-25 µM for SEQ ID No. 3, whereas conjugate of Example 8 has IC50 values in nanomoles.

## Claims

1. A recombinant glycan binding protein comprising a variant of Sclerotium rolfsii Lectin (SRL) represented by SEQ ID NO: 1, wherein, as compared to SEQ ID NO:1, the variant comprises:
a) an additional cysteine at amino acid position 142 at the carboxy-terminal end; or an additional serine at amino acid position 142 and an additional cysteine at amino acid position 143 at the carboxy-terminal end;
b) Serine in place of threonine at amino acid position 1; and
c) Glycine in place of Cysteine at amino acid position 76;
wherein the variant retains binding affinity towards one or more antigens selected from Thomsen-Friedenreich (Galβ1-3GalNAcα-O-Ser/Thr, TF or T) antigen, O-GalNAc Core1 (T antigen), Core2, 'α2,3/6-sialy1 Core1' (Sialyl-T antigen), 'α2,6/6-sialyl Core2' and modified forms of TF and T antigens,
and wherein the amino acid sequence of the said variant has at least 90% sequence identity with SEQ ID NO: 1.

2. The recombinant glycan binding protein as claimed in claim 1, wherein amino acid sequence of the said variant has at least 95% sequence identity with SEQ ID NO: 1.

3. The recombinant glycan binding protein as claimed in claim 1, wherein the variant is devoid of cysteine (C) at amino acid positions between 1 and 141 of SEQ ID NO: 1.

4. The recombinant glycan binding protein as claimed in claim 1, wherein the protein is represented by SEQ ID NO. 3 or SEQ ID NO. 4.

5. The recombinant glycan binding protein as claimed in claim 1, wherein the said protein is conjugated to an agent.

6. The recombinant glycan binding protein as claimed in claim 5, wherein the agent selected from a group comprising of therapeutic agent, cytotoxic agent, radioactive agent, anti-cancer agent, diagnostic agent, and combinations thereof.

7. The recombinant glycan binding protein as claimed in claim 5, wherein the protein is used for targeted delivery of agent.

8. The recombinant glycan binding protein as claimed in claim 1, wherein the protein is for use in the diagnosis or therapeutic treatment of cancer.

9. A pharmaceutical composition comprising:
recombinant glycan binding protein of claim 1; and
one or more pharmaceutically acceptable excipient(s).

10. A recombinant polynucleotide that encodes the protein of claim 1.

11. A cell transfected with the recombinant polynucleotide of claim 10.

## Patentansprüche

1. Rekombinantes Glykan-bindendes Protein, das eine Variante des Sclerotium rolfsii-Lektins (SRL), dargestellt durch SEQ ID Nr. 1 umfasst, wobei im Vergleich zu SEQ ID Nr.1 die Variante umfasst:
a) ein zusätzliches Cystein an Aminosäureposition 142 am Carboxy-Terminus; oder ein zusätzliches Serin an Aminosäureposition 142 und ein zusätzliches Cystein an Aminosäureposition 143 am Carboxy-Terminus;
b) Serin anstelle von Threonin an Aminosäureposition 1; und
c) Glycin anstelle von Cystein an Aminosäureposition 76;
wobei die Variante Bindungsaffinität zu einem oder mehreren Antigenen beibehält, ausgeählt aus Thomsen-Friedenreich- (Galβ1-3GalNAcα-O-Ser/Thr, TF- oder T) -Antigen, O-GalNAc Core1 (T-Antigen), Core2, 'α2,3/6-Sialyl Core1' (Sialyl-T-Antigen), 'α2,6/6-Sialyl Core2' und modifizierten Formen von TF- und T-Antigenen,
und wobei die Aminosäuresequenz der genannten Variante mindestens 90 % Sequenzidentität mit SEQ ID Nr. 1 aufweist.

2. Rekombinantes Glykan-bindendes Protein nach Anspruch 1, wobei die Aminosäuresequenz der genannten Variante mindestens 95% Sequenzidentität mit SEQ ID Nr. 1 aufweist.

3. Rekombinantes Glykan-bindendes Protein nach Anspruch 1, wobei die Variante an Aminosäurepositionen zwischen 1 und 141 der SEQ ID Nr. 1 kein Cystein (C) aufweist.

4. Rekombinantes Glykan-bindendes Protein nach Anspruch 1, wobei das Protein durch SEQ ID Nr. 3 oder SEQ ID Nr. 4 repräsentiert wird.

5. Rekombinantes Glykan-bindendes Protein nach Anspruch 1, wobei das Protein an einen Wirkstoff konjugiert ist.

6. Rekombinantes Glykan-bindendes Protein nach Anspruch 5, wobei der Wirkstoff ausgewählt ist aus einer Gruppe bestehend aus therapeutischem Wirkstoff, zytotoxischem Wirkstoff, radioaktivem Wirkstoff, Antikrebsmittel, diagnostischem Wirkstoff und Kombinationen davon.

7. Rekombinantes Glykan-bindendes Protein nach Anspruch 5, wobei das Protein zur gezielten Verabreichung eines Wirkstoffs verwendet wird.

8. Rekombinantes Glykan-bindendes Protein nach Anspruch 1, wobei das Protein zur Diagnose oder therapeutischen Behandlung von Krebs verwendet wird.

9. Pharmazeutische Zusammensetzung, umfassend:
rekombinantes Glykan-bindendes Protein nach Anspruch 1; und
einen oder mehrere pharmazeutisch akzeptable Hilfsstoffe.

10. Rekombinantes Polynukleotid, das das Protein aus Anspruch 1 codiert.

11. Zelle, die mit dem rekombinanten Polynukleotid aus Anspruch 10 transfiziert wurde.

## Revendications

1. Protéine recombinante de liaison aux glycanes comprenant une variante de la lectine de Sclerotium rolfsii (SRL) représentée par SEQ ID NO: 1, dans laquelle, par rapport à SEQ ID NO: 1, la variante comprend :
a) une cystéine supplémentaire à la position 142 de l'acide aminé à l'extrémité carboxy-terminale ; ou une sérine supplémentaire à la position d'acides aminés 142 et une cystéine supplémentaire à la position d'acides aminés 143 à l'extrémité carboxy-terminale ;
b) une sérine à la place de la thréonine à la position d'acides aminés 1; et
c) une glycine à la place de la cystéine à la position d'acides aminés 76 ;
dans laquelle la variante conserve une affinité de liaison envers un ou plusieurs antigènes choisis parmi Thomsen-Friedenreich (Galβ1-3GalNAcα-O-Ser/Thr, antigène TF ou T), O-GalNAc Core1 (antigène T), Core2, 'α2,3/6-sialyl Core1' (Sialyl-antigène T), 'α2,6/6-sialyl Core2' et des formes modifiées des antigènes TF et T,
et dans laquelle la séquence d'acides aminés de ladite variante présente une identité de séquence d'au moins 90 % avec SEQ ID NO: 1.

2. Protéine recombinante de liaison aux glycanes selon la revendication 1, dans laquelle la séquence d'acides aminés de ladite variante présente une identité de séquence d'au moins 95 % avec SEQ ID NO: 1.

3. Protéine recombinante de liaison aux glycanes selon la revendication 1, dans laquelle la variante est dépourvue de cystéine (C) aux positions d'acides aminés entre 1 et 141 de SEQ ID NO: 1.

4. Protéine recombinante de liaison aux glycanes selon la revendication 1, dans laquelle la protéine est représentée par SEQ ID NO: 3 ou SEQ ID NO: 4.

5. Protéine recombinante de liaison aux glycanes selon la revendication 1, dans laquelle ladite protéine est conjuguée à un agent.

6. Protéine recombinante de liaison aux glycanes selon la revendication 5, dans laquelle l'agent est choisi parmi un groupe comprenant un agent thérapeutique, un agent cytotoxique, un agent radioactif, un agent anticancéreux, un agent de diagnostic et des combinaisons de ceux-ci.

7. Protéine recombinante de liaison aux glycanes selon la revendication 5, dans laquelle la protéine est utilisée pour l'administration ciblée d'un agent.

8. Protéine recombinante de liaison aux glycanes selon la revendication 1, dans laquelle la protéine est destinée à être utilisée dans le diagnostic ou le traitement thérapeutique du cancer.

9. Composition pharmaceutique comprenant:
la protéine recombinante de liaison aux glycanes selon la revendication 1; et
un ou plusieurs excipients pharmaceutiquement acceptables.

10. Polynucléotide recombinant codant pour la protéine selon la revendication 1.

11. Cellule transfectée avec le polynucléotide recombinant selon la revendication 10.
